# FASCICULE DE BREVET EUROPEEN

(11) **EP 2 818 047 B1**
(45) Date de publication et mention de la délivrance du brevet: **27.04.2016**
(21) Numéro de dépôt: 14179749.8
(22) Date de dépôt: 15.03.2012
(51) Int. Cl.: A21D 8/04, C12R 1/85, C12Q 1/04, C12N 1/16, C12N 1/18, C12R 1/865

(54) **Amelioration des panifications a fort taux de levure**
Verbesserung der Herstellung von Brotwaren mit hohem Hefegehalt
Improvement to bread-making with a high yeast content

(30) Priorité: 18.03.2011 FR 1152232
(43) Date de publication de la demande: 31.12.2014
(62) Demande divisionnaire de: 12714788.2
(73) Titulaire: Lesaffre et Compagnie, 75001 Paris (FR)
(72) Inventeur: Lejeune, Pascal, 59200 Tourcoing (FR); Bartolucci, Jean-Charles, 59260 Hellemmes (FR)
(74) Mandataire: Cabinet Plasseraud

(56) Documents cités:
- EP-A1- 0 511 108
- GB-A- 2 139 473
- US-A1- 2004 175 831
- MCKINNON C M ET AL: "WINE YEAST PREFERMENT FOR ENHANCING BREAD AROMA AND FLAVOR", CEREAL CHEMISTRY, AMERICAN ASSOCIATION OF CEREAL CHEMISTS. MINNEAPOLIS, US, vol. 73, no. 1, 1 janvier 1996 (1996-01-01), pages 45-50, XP000554597, ISSN: 0009-0352
- BELL P J L ET AL: "Comparison of fermentative capacities of industrial baking and wild-type yeasts of the species Saccharomyces cerevisiae in different sugar media", LETTERS IN APPLIED MICROBIOLOGY, OXFORD, GB, vol. 32, no. 4, 1 avril 2001 (2001-04-01), pages 224-229, XP002332132, ISSN: 1472-765X, DOI: 10.1046/J.1472-765X.2001.00894.X

## Description

### DOMAINE TECHNIQUE

La présente invention concerne le domaine de la panification, en particulier les panifications nécessitant l'utilisation de quantités importantes de levure.

### ARRIERE-PLAN TECHNOLOGIQUE

La levure joue un rôle clé en panification. En particulier, l'activité fermentative de la levure se traduit par un dégagement gazeux qui permet la levée de la pâte.

Certaines panifications entraînent cependant une diminution de l'activité fermentative de la levure, par exemple en raison de stress subis par la levure, tels qu'une forte teneur en sucre dans la pâte ou la surgélation de la pâte avant fermentation. Même en utilisant des levures adaptées à ces stress, il est nécessaire d'utiliser une quantité plus conséquente de levure dans la pâte.

Par ailleurs, il peut être nécessaire d'utiliser une quantité plus importante de levure pour compenser un mode opératoire moins adapté. Par exemple, le dégagement gazeux peut être plus faible en raison de la faiblesse du pétrissage et/ou du manque de puissance calorique des instruments de cuisson. C'est notamment le cas des applications ménagères, lors d'un pétrissage manuel, une cuisson dans un four ménager ou encore une panification en machine à pain. Le dégagement gazeux peut également être plus faible en raison d'un schéma de panification court, c'est-à-dire avec une durée de fermentation très réduite.

Afin de résoudre les problèmes évoqués ci-dessus, des quantités plus importantes de levure sont donc utilisées. Toutefois, ceci présente l'inconvénient de donner des produits de panification développant une note aromatique non souhaitée, appelée par la suite « note levure ».

Les levains, appelés également préferments, sont traditionnellement utilisés en panification pour améliorer les qualités organoleptiques du pain. Certains auteurs se sont intéressés à des préferments obtenus à partir de levures non boulangères.

McKinnon et al. (Cereal Chem. 73(1):45-50, 1996) décrivent ainsi des préferments liquides à base de levures oenologiques. Toutefois, les souches de *Saccharomyces* testées donnent un arôme similaire à la souche de boulangerie témoin et sont écartées par les auteurs. Seules des souches de *Torulaspora delbrueckii* et une souche de *Hansenula anomala* donnent des arômes différents dans le préferment liquide.

Malgré leur intérêt certain pour améliorer les propriétés organoleptiques du pain, l'utilisation des levains présente plusieurs inconvénients, parmi lesquels la durée nécessaire pour les obtenir, leur durée de conservation limitée, l'homogénéité des produits de panification obtenus et/ou le coût du levain s'il est acheté tout prêt. De plus, l'utilisation des levains génère une typicité aromatique, à savoir des notes aromatiques de type « acide » ou « vinaigre », non désirées dans les applications visées.

Le document US004175831 décrit la sélection de souches de levure, par exemple la souche AT25, qui ne donnent pas le goût de levure au pain.

Il existe un réel besoin de fournir un procédé permettant d'obtenir les produits de panification souhaités, mais avec un masquage partiel ou total de la note levure, malgré la présence de stress, tels qu'une teneur en sucre élevée dans la pâte et/ou la surgélation de la pâte, et/ou malgré un mode opératoire moins adapté, par exemple lors des applications ménagères. De plus, un tel procédé ne doit pas modifier les habitudes du consommateur (professionnel ou non), à la fois en terme de durée du procédé, nombre d'étapes et, de préférence, nombre d'ingrédients à utiliser.

### RESUME DE L'INVENTION

La description concerne un procédé de sélection d'une souche de levure permettant d'obtenir des levures appropriées à des panifications à fort taux de levure.
Un premier objet de l'invention concerne une composition apte à donner un produit de panification, comprenant de la levure, de la farine et du sel, caractérisée en ce que ladite levure a une force fermentative sous forme sèche inférieure à 70 ml dans une pâte sans sucre, ladite levure étant obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447.
Un deuxième objet de l'invention concerne un procédé de préparation d'une telle composition apte à donner un produit de panification, comprenant une étape de mélange des ingrédients comprenant de la levure, telle que définie ci-dessus, de la farine et du sel, ladite levure ayant une force fermentative sous forme sèche inférieure à 70 ml dans une pâte sans sucre.
Un troisième objet de l'invention est un procédé de préparation d'un produit de panification comprenant les étapes suivantes :
a) une étape de mélange des ingrédients comprenant levure, farine, sel et eau, ladite levure telle que définie ci-dessus
   sous forme sèche ayant une force fermentative inférieure à 70 ml dans une pâte sans sucre, pour obtenir une pâte,
b) une étape de fermentation de la pâte, pour obtenir une pâte fermentée, et
c) une étape de cuisson de la pâte fermentée pour obtenir un produit de panification.
Un quatrième objet de l'invention concerne l'utilisation d'une levure telle que définie ci-dessus, dont la force fermentative sous forme sèche est inférieure à 70 ml dans une pâte sans sucre, comme agent levant et masquant la note levure.

### BREVE DESCRIPTION DES FIGURES

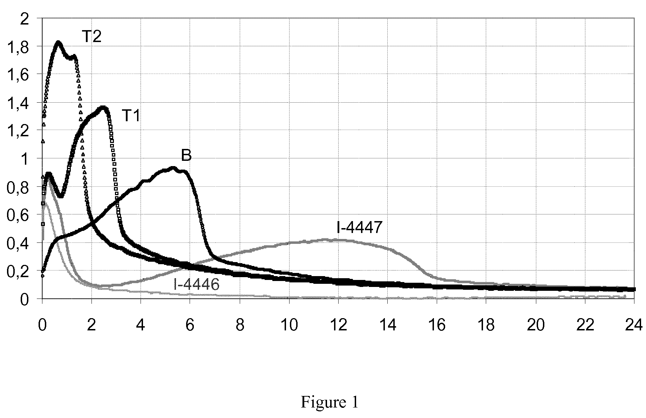

Figure 1 : Profil fermentaire sur 24h à 30°C de levures sous forme sèche (sur pâte contenant 20 g de farine et 320 mg de levure sèche en masse de matière sèche). La vitesse de dégagement gazeux (en ml de CO₂ par minute) est indiquée en fonction du temps (en heures). T1 (courbe avec carrés) est une levure de boulangerie utilisée traditionnellement dans des pâtes sucrées. T2 (courbe avec triangles) est une levure de boulangerie utilisée traditionnellement dans des pâtes sans sucre. B (courbe avec cercles) est une levure de brasserie. La vitesse de dégagement gazeux obtenue avec la levure issue de la souche I-4446 est représentée par un trait fin gris et celle de la levure issue de la souche I-4447 par un trait épais gris.

### DESCRIPTION DETAILLEE DE L'INVENTION

Les Inventeurs ont mis en évidence de manière surprenante et inattendue que certaines souches de levure qui ne sont pas utilisées dans des applications de boulangerie donnent des levures qui peuvent être utilisées à la fois comme agent levant et agent de masquage de la note levure dans des panifications utilisant une forte dose de levure.

Les levures issues des souches de levure selon l'invention apportent ainsi une solution simple aux problèmes évoqués ci-dessus, en se substituant à la levure de boulangerie traditionnelle, sans modifier les caractéristiques du produit de panification et sans entraîner d'autres modifications du procédé de panification, hormis éventuellement une quantité de levure utilisée plus importante.

Ainsi, les habitudes du consommateur (le professionnel ou non) ne sont pas modifiées en termes de durée du procédé, nombre d'étapes, nombre et type d'ingrédients à utiliser.

La solution apportée par les levures selon l'invention permet de plus d'éviter l'utilisation de levain, préferment ou starter.

Un autre avantage des levures selon l'invention est qu'elles peuvent se présenter sous le même format que la levure de boulangerie traditionnelle, par exemple sous forme de levure liquide, levure pressée ou levure sèche.

Il n'y a donc pas de modification de la durée de conservation de l'ingrédient de base, à savoir la levure, pour le consommateur.

Le masquage de la note levure peut être partiel ou total.

Par « masquage partiel de la note levure », on entend une diminution de la note levure dans le produit de panification.

Par « masquage total de la note levure », on entend l'absence de note levure dans le produit de panification.

Le terme « masquage » est utilisé ici de manière large.

Le masquage de la note levure peut en effet être obtenu par une concentration diminuée ou une disparition, dans le produit de panification, d'au moins une molécule aromatique contribuant à la note levure et/ou par l'apparition d'au moins une molécule qui masque partiellement ou totalement la note levure.

Un masquage partiel de la note levure se traduit notamment par une diminution de l'arôme levure et/ou de l'odeur levure des produits de panification.

Les expressions « masquer partiellement la note levure » et « diminuer la note levure » sont ici synonymes.

Un masquage total de la note levure se traduit par la disparition de l'arôme levure et de l'odeur levure dans les produits de panification.

L'arôme levure et l'odeur levure peuvent être évalués en analyse sensorielle ou par mesure chimique, comme indiqué ci-après.

En l'absence de stress dans la pâte, en particulier dans le cas d'une pâte sans sucre et dans un protocole adapté, la levure de boulangerie est utilisée à environ 0,7% de levure, le pourcentage étant exprimé en masse de matière sèche sur la masse de farine.

Une pâte sans sucre désigne ici une pâte dans laquelle il n'a pas été ajouté de sucre.

Dans une pâte sans sucre, les sucres présents proviennent de la farine.

Des panifications utilisant une forte dose de levure sont définies ici comme des panifications utilisant plus de 0,7% de levure, de préférence au moins 0,9% de levure, les pourcentages étant exprimés en masse de matière sèche sur la masse de farine.

Les expressions « forte dose de levure » et « quantité importante de levure » sont ici synonymes.

Cette différence dans la quantité de levure utilisée peut sembler petite, mais elle est suffisante pour conférer une note levure aux produits de panification obtenus avec au moins 0,9% de levure, le pourcentage étant exprimé en masse de matière sèche sur la masse de farine. L'expression « pourcentage exprimé en masse sur la masse de farine » est synonyme ici de « pourcentage du boulanger ».

Le pourcentage du boulanger se réfère toujours à la masse d'un ingrédient donné d'une pâte, par rapport à la masse totale de la farine dans ladite pâte.

La levure pouvant être sous la forme d'une levure liquide, d'une levure pressée ou d'une levure sèche, les pourcentages de levure sont exprimés en masse de matière sèche levure sur la masse de farine.

De manière surprenante, les levures selon l'invention permettent un masquage de la note levure, alors même qu'une quantité de levure plus importante est utilisée.

Une quantité plus importante de levure est utilisée afin d'obtenir une activité fermentative équivalente à la levure de boulangerie traditionnelle.

Par « levure de boulangerie traditionnelle », on entend ici la levure de *Saccharomyces cerevisiae* habituellement utilisée dans une panification donnée.

Une panification donnée est définie ici par une recette et un protocole de panification.

La recette indique la quantité de chaque ingrédient utilisé.

Le protocole de panification indique les paramètres des différentes étapes de la panification. L'expression « activité fermentative équivalente » signifie que le dégagement gazeux dans la pâte est équivalent.

Une activité fermentative équivalente se traduit en particulier par un volume spécifique du produit de panification équivalent.

Le volume spécifique d'un produit de panification est équivalent à celui d'un produit de panification préparé avec une levure de boulangerie traditionnelle, si l'écart entre leur volume spécifique est inférieur à + ou -10%.

Le volume spécifique d'un produit de panification est défini comme le volume du produit de panification rapporté à sa masse (en cm³/g).

L'activité fermentative d'une levure peut être évaluée en mesurant sa force fermentative.

La force fermentative d'une levure correspond au volume de CO₂ (en ml) produit par la levure en fermentation dans une pâte.

L'expression « souche de levure » désigne une population relativement homogène de cellules de levure.

Une souche de levure est obtenue à partir de l'isolement d'un clone, un clone étant une population de cellules obtenue à partir d'une seule cellule de levure.

Une levure est obtenue par culture d'une souche de levure, c'est-à-dire par ensemencement d'une quantité d'une souche de levure dans un milieu de culture, puis culture dans des conditions permettant la multiplication des cellules de levure.

Les Inventeurs ont mis au point un procédé de sélection original d'une souche de levure permettant d'obtenir des levures appropriées à des panifications à fort taux de levure.

La description a ainsi pour objet un procédé de sélection d'une souche de levure donnant une levure masquant totalement ou partiellement la note levure, comprenant les étapes suivantes :
- une étape de culture d'une souche de levure à tester, pour obtenir une levure à tester,
- une étape de préparation d'un produit de panification témoin à partir d'une pâte comprenant farine, sel, eau et au moins 0,9% d'une levure de boulangerie, le pourcentage étant exprimé en masse de matière sèche sur la masse de farine,
- une étape de préparation d'un produit de panification à tester identique à l'étape de préparation du produit de panification témoin, exceptée que la levure est la levure à tester dans une quantité permettant d'obtenir un volume spécifique équivalent à celui du produit de panification témoin,
- une étape de sélection d'au moins une levure masquant partiellement ou totalement la note levure dans le produit de panification à tester par comparaison avec le produit de panification témoin, et
- une étape de sélection de la souche de levure correspondant à la levure masquant partiellement ou totalement la note levure dans le produit de panification à tester.

Les souches de levure à tester sont de préférence des souches de *Saccharomyces.*

Les souches de *Saccharomyces* sont telles que définies dans l'ouvrage de référence The yeasts, a taxonomic study, de Kurtzman et Fell, 14^{ème} édition, 1998.

Les souches de Saccharomyces comprennent en particulier les espèces *cerevisiae, exigus* et *bayanus*.

Les souches de levure testées ne sont pas des souches de levure de boulangerie traditionnelle, et plus généralement ne sont pas des souches de levure utilisées comme agent levant en panification.

Les souches de levure testées sont par exemple des souches de levure destinées à la production d'alcool, tel que l'alcool dit « de bouche », destiné à la fabrication de boissons alcoolisées, et/ou l'alcool industriel, destiné par exemple aux biocarburants ou aux industries chimiques, des souches de levure oenologiques (également appelées souches de levure de vinification), ou des souches de levure de collection qui ne sont pas utilisées dans des applications de boulangerie, distillerie, brasserie ou vinification.

La levure testée peut être sous la forme d'une levure liquide, d'une levure pressée ou d'une levure sèche.

La levure testée est de préférence sous la forme d'une levure sèche.

La levure de boulangerie utilisée dans le procédé de sélection pour préparer le produit de panification témoin est une levure de boulangerie traditionnelle telle que définie ci-dessus. La levure testée et la levure de boulangerie utilisée dans le produit de panification témoin sont sous la même forme, de préférence sous forme sèche.

La description a également pour objet un procédé de sélection tel que défini ci-dessus, dans lequel le produit de panification à tester est préparé à partir d'une pâte comprenant au moins 1,3% de levure à tester, le pourcentage étant en masse de matière sèche sur la masse de farine.

Dans un mode de réalisation préféré, la présente invention a pour objet un procédé de sélection tel que défini ci-dessus, dans lequel le produit de panification à tester est préparé à partir d'une pâte comprenant de 0,9% à 3,2% de la levure à tester, de préférence de 1,1% à 1,9% de la levure à tester, le pourcentage étant en masse de matière sèche sur la masse de farine.

La pâte du produit de panification à tester comprend donc la levure à tester dans une quantité permettant d'obtenir un volume spécifique égal à plus ou moins 10% du volume spécifique du produit de panification témoin.

La description a pour objet un procédé de sélection tel que défini ci-dessus, dans lequel l'étape de préparation d'un produit de panification comprend les sous-étapes suivantes :
- mélange des ingrédients comprenant la farine, le sel, l'eau et la levure, pour obtenir une pâte,
- éventuellement surgélation de la pâte ou blocage de la pâte à une température inférieure à 15°C,
- fermentation de la pâte, pour obtenir une pâte fermentée, et
- cuisson de la pâte fermentée pour obtenir un produit de panification.

Le blocage de la pâte est une étape intermédiaire de stockage de la pâte à une température inférieure à 15°C.

La présente invention a également pour objet un procédé de sélection tel que défini ci-dessus, dans lequel l'étape de préparation du produit de panification comprend comme ingrédient au moins 10% de sucre, de préférence au moins 15% de sucre, le pourcentage étant exprimé en masse sur la masse de farine.

Dans un mode de réalisation préféré, la présente invention a pour objet un procédé de sélection tel que défini ci-dessus, dans lequel l'étape de préparation du produit de panification comprend comme ingrédient 15% à 25% de sucre, le pourcentage étant exprimé en masse sur la masse de farine.

Le sucre est par exemple du saccharose, du glucose, du fructose ou un mélange de ceux-ci.

Dans un mode de réalisation particulier, le procédé de sélection tel que défini ci-dessus, caractérisé en ce qu'au moins l'une des sous-étapes de l'étape de préparation d'un produit de panification est réalisée en machine à pain.

Dans un mode de réalisation avantageux, le procédé de sélection tel que défini ci-dessus, caractérisé en ce que toutes les sous-étapes de l'étape de préparation d'un produit de panification sont réalisées en machine à pain.

Dans un mode de réalisation particulier, le procédé de sélection tel que défini ci-dessus, caractérisé en ce que l'étape de préparation d'un produit de panification est effectuée selon un schéma court.

Un schéma court signifie que la durée entre le début de l'étape de mélange et la fin de l'étape de cuisson est inférieure ou égale à 3h, de préférence inférieure ou égale à 2h30, de préférence inférieure ou égale à 2h.

L'étape de sélection d'au moins une levure masquant totalement ou partiellement la note levure dans le produit de panification à tester peut être effectuée par tout moyen approprié, tel que l'analyse sensorielle, une mesure chimique, ou l'analyse sensorielle couplée à une mesure chimique.

L'analyse sensorielle consiste par exemple à évaluer la note levure dans le produit de panification et à comparer le résultat obtenu avec celui du produit de panification témoin préparé avec une levure de boulangerie traditionnelle.

L'évaluation de la note levure est de préférence effectuée par un panel d'experts.

Un panel étudie de manière ordonnée et structurée les propriétés d'un produit afin de le décrire, classer ou améliorer d'une façon objective et rigoureuse.

Les experts sont des sujets qualifiés qui ont une bonne acuité sensorielle, qui sont entraînés à l'utilisation des méthodes d'évaluation sensorielle et qui sont capables d'effectuer de façon fiable tous les types d'essais. Les performances des experts sont de préférence contrôlées régulièrement.

L'évaluation de la note levure peut se faire en évaluant l'arôme levure et/ou l'odeur levure. L'évaluation peut se faire en attribuant un chiffre, par exemple sur une échelle de 0 à 10, en fonction de l'intensité de la note levure, par exemple en fonction de l'intensité de l'arôme levure et/ou de l'odeur levure.

L'intensité maximale (chiffre 10) attribuée à l'arôme levure ou à l'odeur levure correspond respectivement à l'arôme levure et odeur levure d'une baguette obtenue à partir d'une pâte crue surgelée puis cuisson, ladite baguette étant préparée avec 6% (en masse sur la masse de farine) de levure de boulangerie traditionnelle à 32% de matière sèche (par exemple l'Hirondelle bleue).

Une levure est alors sélectionnée si le chiffre attribué au produit de panification à tester préparé avec ladite levure pour la note levure, c'est-à-dire pour l'arôme levure et/ou l'odeur levure, est inférieur à celui du produit de panification témoin.

La mesure chimique consiste, dans un premier temps, à identifier au moins une molécule aromatique correspondant à la note levure dans le produit de panification témoin, puis à comparer la quantité de ladite molécule aromatique dans le produit de panification à tester par rapport à la quantité de ladite molécule aromatique dans le produit de panification témoin.

Le terme « identifier au moins une molécule aromatique » signifie identifier ladite molécule aromatique structurellement et/ou analytiquement.

La molécule aromatique peut par exemple être identifiée par un pic particulier obtenu par chromatographie en phase gazeuse couplée à une spectrométrie de masse ou une olfactométrie.

L'identification et l'évaluation de la quantité d'une molécule aromatique peut se faire par exemple par extraction des arômes de la mie du produit de panification par la technique dite de « Purge and trap », telle que décrite dans Madrera et al. (Journal of Chromatography, 2005, 245-251), puis chromatographie en phase gazeuse suivie d'une spectrométrie de masse et/ou d'une olfactométrie.

La quantité de la molécule aromatique peut être mesurée quantitativement ou qualitativement.

Par exemple, on peut comparer l'intensité du pic correspondant à la molécule aromatique dans le produit de panification à tester et dans le produit de panification témoin.

Dans un mode de réalisation avantageux, l'identification d'au moins une molécule aromatique correspondant à la note levure dans le produit de panification témoin est effectuée sur au moins deux produits de panification témoin, de préférence au moins trois produits de panification témoin, préparés avec des quantités croissantes de levure de boulangerie.

Une levure est alors sélectionnée si la quantité de ladite molécule aromatique mesurée dans le produit de panification à tester préparé avec ladite levure est inférieure à celle mesurée dans le produit de panification témoin.

Dans ce qui précède et ce qui suit, un produit de panification témoin est obtenu selon le même protocole et la même recette que le produit de panification testé, mise à part la levure utilisée et sa quantité. En particulier, le produit de panification témoin utilise une levure sous la même forme que la levure testée (par exemple forme sèche, pressée ou liquide).

La description a également pour objet les souches de levure obtenues par le procédé de sélection tel que défini ci-dessus.

Les souches de levure selon la description ne sont pas des souches de levure de boulangerie traditionnelle, et plus généralement ne sont pas des souches de levure utilisées comme agent levant en panification.

Les levures obtenues par culture des souches de levure selon la description permettent de masquer totalement ou partiellement la note levure dans les produits de panification, en particulier dans les produits de panification préparés avec une forte dose de levure.

De manière surprenante et inattendue, les Inventeurs ont mis en évidence que les levures obtenues par culture des souches de levure selon la description ont un profil fermentaire bien particulier.

Ainsi, les levures selon la description sont caractérisées par une force fermentative, sous forme sèche, inférieure à 70 ml dans une pâte sans sucre, de préférence inférieure à 60 ml dans une pâte sans sucre.

La force fermentative est mesurée dans une pâte contenant 20g de farine sur une durée de 2 heures à 30°C, tel que défini ci-dessous.

La force fermentative des levures selon la description dans une pâte sans sucre est très inférieure à la fois à celle d'une levure adaptée aux pâtes sans sucre, mais également très inférieure à celle d'une levure adaptée aux pâtes sucrées et qui n'est donc pas très performante sur pâte sans sucre.

Par ailleurs, les levures selon la description sont caractérisées par une force fermentative, sous forme sèche, inférieure à 90 ml, de préférence inférieure 80 ml dans une pâte sucrée à 10%.

La force fermentative des levures selon la description dans une pâte sucrée à 10% est très inférieure à celle d'une levure adaptée aux pâtes sucrées, mais également à celle d'une levure adaptée aux pâtes sans sucre et qui n'est donc pas très performante sur pâte sucrée.

Enfin, les levures selon la description sont caractérisées par une force fermentative, sous forme sèche, inférieure à 70 ml dans une pâte sucrée à 20%, donc très inférieure à celle d'une levure adaptée aux pâtes sucrées.

La force fermentative est mesurée selon des techniques classiques connues de l'homme du métier, adaptées du protocole décrit par Burrows et Harrison dans « Journal of the Institute of Brewing », Vol 65, 1959.

La force fermentative est notamment mesurée au moyen d'un fermentomètre ou d'un Risograph^{®} (*National Manufacturing, Lincoln, Nebraska*).

La force fermentative est mesurée ici sur une pâte constituée de farine et d'une levure sèche, sur une durée de 2 heures (*cf. exemple 1*).

La force fermentative est mesurée dans une pâte sans sucre et dans des pâtes sucrées à 10% et 20% (pourcentages exprimés en masse sur la masse de farine).

La force fermentative d'une levure dans une pâte sans sucre est mesurée ici dans une pâte contenant 20g de farine.

La force fermentative d'une levure dans une pâte sucrée à 10% ou 20% est mesurée ici dans une pâte contenant 20g de farine et respectivement 2g ou 4g de saccharose.

La levure sèche, dans une quantité égale à 160 mg de matières sèches, est réhydratée, puis mise en suspension dans une solution aqueuse contenant 27 g/l de NaCl (cf. exemple 1).

La farine (avec ou sans saccharose) et ladite suspension de levures sont ensuite malaxées pendant 40 secondes dans un pétrin, de manière à obtenir une pâte qui est ensuite placée dans un récipient mis au bain-marie à 30°C. 13 minutes après le début du malaxage, le récipient contenant la pâte est fermé hermétiquement.

Le volume de dégagement gazeux est mesuré au cours de la 1^{ère} heure et au cours de la 2^{ème} heure, au moyen d'un Risograph^{®} (*National Manufacturing, Lincoln, Nebraska*). On indique ensuite le volume total en ml sur 2 heures à 30°C.

Enfin, de manière surprenante, les Inventeurs ont montré que les levures selon la description permettent non seulement de masquer totalement ou partiellement la note levure, mais également d'apporter une note positive absente dans le produit de panification témoin et qui diffère selon la panification.

Le procédé de sélection selon la description a ainsi permis de sélectionner les trois souches de levure suivantes : la souche déposée auprès de la CNCM sous le numéro I-4445, la souche déposée auprès de la CNCM sous le numéro I-4446 et la souche déposée auprès de la CNCM sous le numéro I-4447.

Les souches de levure I-4445, I-4446 et I-4447 ont été déposées en vertu du traité de Budapest le 9 février 2011 auprès de la CNCM (*Collection Nationale de Cultures de Microorganismes*), 25 rue du Docteur Roux, 75724 Paris cedex 15, France.

La souche de levure I-4445 est une souche de distillerie, habituellement utilisée pour la production d'alcool.

La souche de levure I-4445 est une souche de *Saccharomyces cerevisiae.*

La levure obtenue par culture de la souche de levure I-4445 a une force fermentative, sous forme sèche, inférieure à 60 ml dans une pâte sans sucre, inférieure à 70 ml dans une pâte sucrée à 10% et inférieure à 50 ml dans une pâte sucré à 20% (cf. exemple 1).

La levure obtenue par culture de la souche de levure I-4445 permet de diminuer la note levure de produits de panification contenant des quantités importantes de levure, en particulier dans des panifications en machine à pain, dans des pâtes sucrées, dans des pâtes crues surgelées et/ou dans des panifications selon un schéma court (cf. exemple 2).

La levure obtenue par culture de la souche de levure I-4445 permet également d'apporter une note positive dans plusieurs types de panification (cf. exemple 2), telle que :
- une odeur et un arôme amande, par exemple dans un pain brioché réalisé en machine à pain,
- une odeur et un arôme blé mûr, par exemple dans des pâtes sans sucre, telle qu'une baguette obtenue en schéma direct,
- une odeur et un arôme rhum, par exemple dans une brioche obtenue à partir d'une pâte crue surgelée ou un rotimani, et
- une saveur sucrée, par exemple dans des pâtes très sucrées, telle que celle du rotimani. Le rotimani est un pain de mie indonésien obtenu à partir d'une pâte contenant une très forte teneur en sucre.

La souche de levure I-4446 est une souche de levure de la collection du Demandeur qui n'est pas utilisée en distillerie, oenologie, brasserie, ou boulangerie en tant qu'agent levant.

La souche de levure I-4446 est une souche de *Saccharomyces cerevisiae*.

La levure obtenue par culture de la souche de levure I-4446 a une force fermentative, sous forme sèche, inférieure à 45 ml dans une pâte sans sucre, inférieure à 80 ml dans une pâte sucrée à 10% et inférieure à 65 ml dans une pâte sucré à 20% (cf. exemple 1).

La levure obtenue par culture de la souche de levure I-4446 permet de diminuer la note levure de produits de panification contenant des quantités importantes de levure, en particulier dans des panifications en machine à pain, dans des pâtes sucrées, dans des pâtes crues surgelées et/ou dans des panifications selon un schéma court (cf. exemple 2).

La levure obtenue par culture de la souche de levure I-4446 permet également d'apporter une note positive dans plusieurs types de panification (cf. exemple 2), telle que :
- une odeur et un arôme rhum, par exemple dans des pâtes sucrées, comme dans un pain brioché réalisé en machine à pain, dans une brioche obtenue à partir d'une pâte crue surgelée ou un rotimani, et
- une saveur sucrée, par exemple dans des pâtes très sucrées, telles que celle du rotimani.

La souche de levure I-4447 est une souche d'oenologie, habituellement utilisée en vinification.

La souche de levure I-4447 est une souche de *Saccharomyces bayanus.*

La levure obtenue par culture de la souche de levure I-4447 a une force fermentative, sous forme sèche, inférieure à 55 ml dans une pâte sans sucre, inférieure à 80 ml dans une pâte sucrée à 10% et inférieure à 70 ml dans une pâte sucré à 20% (cf. exemple 1).

La levure obtenue par culture de la souche de levure I-4447 permet de diminuer la note levure de produits de panification contenant des quantités importantes de levure, en particulier dans des panifications en machine à pain, dans des pâtes sucrées, dans des pâtes crues surgelées et/ou dans des panifications selon un schéma court (cf. exemple 2).

La levure obtenue par culture de la souche de levure I-4447 permet également d'apporter une note positive dans plusieurs types de panification (cf. exemple 2), telle que :
- une odeur et un arôme amande, par exemple dans un pain brioché réalisé en machine à pain,
- une odeur et un arôme blé mûr, par exemple dans une pâte sans sucre telle qu'une baguette obtenue en schéma direct,
- une saveur sucrée et/ou un arôme rhum, par exemple dans des pâtes très sucrées, telles que celle du rotimani.

La présente description a également pour objet toutes les souches dérivées des souches de levure selon la description et qui partagent les mêmes propriétés de masquage total ou partiel de la note levure.

La présente description a plus particulièrement pour objet des souches dérivées des souches de levure selon la description lesdites souches dérivées donnant des levures ayant une force fermentative inférieure à 70 ml, sous forme sèche, dans une pâte sans sucre. La force fermentative est tel que mesuré ci-dessus.

Par l'expression « souche dérivée », on désigne une souche dérivée par toute transformation quelle qu'elle soit, comme par exemple un ou plusieurs croisements et/ou une ou plusieurs mutations et/ou une ou plusieurs transformations génétiques.

Une souche dérivée par croisement peut être obtenue par croisement d'une souche selon la description avec la même souche, ou une autre souche selon la description ou une autre souche quelconque.

Une souche dérivée par mutation peut être une souche qui a subi au moins une mutation spontanée dans son génome ou au moins une mutation induite, par exemple par mutagenèse. La ou les mutations de la souche dérivée sont silencieuses ou non.

Par l'expression « mutagenèse », on désigne à la fois la mutagenèse classique obtenue par rayonnements ou par des agents chimiques mutagènes, et la mutagenèse insertionnelle par transposition ou par intégration d'un fragment d'ADN exogène.

La mutagenèse par rayonnements comprend l'utilisation de rayonnements UV, X, ou gamma. Les agents chimiques mutagènes sont par exemple l'EMS (éthyl-méthyl sulfonate), l'EES (éthyl-éthyl sulfonate), la nitrosoguanidine, l'acide nitreux, l'aflatoxine B1, l'hydroxylamine, la 5-bromo uracile, la 2-amino purine, la proflavine et/ou l'acridine orange.

Une souche dérivée par transformation génétique est une souche dans laquelle a été introduit un ADN exogène.

Ledit ADN exogène peut être apporté par un plasmide.

Ledit ADN exogène est de préférence intégré dans le génome de la levure.
la description a également pour objet un procédé de transformation d'une souche de levure selon la description, pour obtenir une souche dérivée telle que définie ci-dessus, ledit procédé de transformation comprenant une étape de transformation de ladite souche par au moins un croisement et/ou au moins une mutation et/ou au moins une transformation génétique.

La présente description concerne également des souches de levure susceptibles d'être obtenues par le procédé de transformation tel que défini ci-dessus.

La présente description a également pour objet une levure obtenue par culture d'une souche de levure telle que définie ci-dessus ou par culture d'une souche de levure dérivée telle que définie ci-dessus.

Une levure selon la description est obtenue par culture d'une souche de levure selon la description notamment comme décrit dans le livre de référence « Yeast Technology », 2ème édition, 1991, G. Reed et T.W. Nagodawithana, publié par Van Nostrand Reinhold, ISBN 0-442-31892-8.

L'obtention d'une levure selon la description à l'échelle industrielle comprend en général au moins les deux premières étapes de l'ensemble des étapes suivantes :
- multiplication d'une souche de levure dans un milieu de culture en plusieurs stades, d'abord en semi-anaérobiose, puis en aérobiose,
- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,
- filtration de la crème de levure liquide ainsi obtenue, en général sur un filtre rotatif sous vide, pour obtenir une levure fraîche déshydratée contenant de 26% à 35% de matière sèche,
- malaxage de ladite levure fraîche déshydratée, pour obtenir une masse homogène,
- extrusion de la levure ainsi obtenue, pour obtenir :
   - une levure pressée sous forme de pains de levure fraîche ou de levure fraîche émiettée, contenant environ 30% de matière sèche, ou
   - une levure sous forme de particules, en général de granules, si la levure est destinée à être séchée,
- éventuellement, séchage de manière ménagée, dans un courant d'air chaud, par exemple par fluidisation, des particules de levures obtenues par extrusion pour obtenir de la levure sèche.

L'étape de séchage est de préférence un séchage rapide ménageant en présence d'un émulsifiant.

Parmi les émulsifiants pouvant être utilisés lors de l'étape de séchage, on peut choisir le monostéarate de sorbitan, utilisé par exemple à une concentration d'environ 1,0% (en poids sur le poids de levure sèche).

Les levures selon la description peuvent être utilisées sous toute forme possible.

Par exemple, la présente description a pour objet une levure telle que définie ci-dessus, caractérisée en ce qu'elle est sous forme de crème de levure, de levure pressée, de levure sèche ou de levure surgelée.

Les levures fraîches sont caractérisées par une teneur élevée en eau en comparaison aux levures sèches. Les levures fraîches englobent les crèmes de levure et les levures pressées. Les crèmes de levure, appelées également « levures liquides », sont des suspensions aqueuses de cellules de levure présentant une viscosité de type crème.

Par crème de levure, on comprend une suspension liquide, typiquement une suspension aqueuse, de cellules vivantes de levure, ladite suspension ayant une teneur en matière sèche d'au moins 12% en masse, généralement comprise d'environ 12 à environ 50% en masse (définition étendue de crème de levure).

De préférence, la crème de levure répond à la définition au sens strict, c'est-à-dire qu'elle présente une teneur en matière sèche comprise d'environ 12 à environ 25% en masse, de préférence d'environ 14 à environ 22% en masse.

Parmi les levures pressées, on distingue les levures pressées en bloc compact, appelées également « pains de levure » qui sont caractérisées par une teneur en matière sèche comprise d'environ 26% à environ 35%, et les levures pressées en granules qui sont caractérisées par une teneur en eau comprise d'environ 21% à environ 35%.

Les levures sèches sont caractérisées par une teneur en matière sèche supérieure à environ 92%.

Les levures surgelées sont caractérisées par une teneur en matière sèche comprise d'environ 74% à environ 80%.

La présente invention a également pour objet une composition apte à donner un produit de panification, comprenant une levure obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447, de la farine et du sel.

La levure selon la description est une levure telle que définie ci-dessus. En particulier, la levure selon la description permet de masquer partiellement ou totalement la note levure de produits de panification utilisant une forte dose de levure.

Une levure selon la description est appelée, pour simplifier, de la manière suivante « levure masquant totalement ou partiellement la note levure ».

Une levure préférée selon la description permet non seulement de masquer totalement ou partiellement la note levure, mais également d'apporter une note positive absente dans le produit de panification obtenu avec une levure de boulangerie traditionnelle.

La présente invention a plus particulièrement pour objet une telle que définie ci-dessus apte à donner un produit de panification, comprenant une levure, de la farine et du sel, caractérisée en ce que ladite levure est obtenue par culture d'une souche de levure selon l'invention.

Une souche de levure selon la description est une souche de levure telle que définie ci-dessus.

L'expression « apte à donner un produit de panification » signifie que, mise à part éventuellement l'eau, la composition contient les ingrédients essentiels pour obtenir une pâte. En particulier, l'expression « apte à donner un produit de panification » exclut que la composition boulangère selon l'invention soit un levain ou un starter.

Un levain, appelé également préferment, est un produit générant ou contenant un ou plusieurs acides organiques et obtenu par fermentation d'un substrat au moyen d'une biomasse, ledit substrat contenant majoritairement de la farine, ladite biomasse contenant au moins une bactérie.

La ou les bactéries peuvent provenir de la farine.

La ou les bactéries sont de préférence des bactéries lactiques.

Un levain peut être un levain sec, un levain pâteux ou un levain liquide.

Le levain peut être obtenu à partir d'un starter.

Un starter est une composition, généralement sous forme de concentré ou de crème, contenant une ou plusieurs espèces de bactéries lactiques.

Les levains secs sont également appelés farine fermentée, farine fermentée déshydratée, farine préfermentée, levain déshydraté.

Une composition selon l'invention peut comprendre un levain et/ou un starter, en plus d'une levure selon l'invention, de la farine et du sel.

Toutefois, dans un mode de réalisation préféré, la composition selon l'invention ne comprend pas de levain ou de starter, et plus généralement aucune préparation obtenue après une phase de fermentation.

Un produit de panification selon l'invention est, de préférence, un produit de panification obtenu par cuisson au four d'une pâte fermentée par une levure.

Un produit de panification selon l'invention est par exemple choisi parmi pain, brioche, pain de mie, viennoiseries.

Le four peut être n'importe quel type de four, y compris une machine à pain.

Dans un mode de réalisation préféré, le four n'est pas un four à micro-ondes.

Dans un mode de réalisation avantageux, le produit de panification selon l'invention n'est pas un produit de panification obtenu par cuisson d'une pâte fermentée à la vapeur ou en friture, par exemple pour obtenir un beignet.

Dans un autre mode de réalisation avantageux, le produit de panification selon l'invention ne comprend pas les tartes, pizzas, gâteaux, biscuits.

La présente invention a plus particulièrement pour objet une composition, telle que définie ci-dessus, apte à donner un produit de panification, comprenant une levure, de la farine, et du sel, caractérisée en ce que ladite levure a une force fermentative sous forme sèche inférieure à 70 ml dans une pâte sans sucre, de préférence inférieure à 60 ml dans une pâte sans sucre.

La force fermentative est mesurée dans une pâte contenant 20g de farine sur une durée de 2 heures à 30°C, tel que défini ci-dessus.

La présente invention a également pour objet une composition apte à donner un produit de panification, telle que définie ci-dessus, caractérisée en ce que ladite levure sous forme sèche a une force fermentative inférieure à 90 ml dans une pâte sucrée à 10%, de préférence inférieure à 80 ml dans une pâte sucrée à 10%.

La présente invention a également pour objet une composition apte à donner un produit de panification, telle que définie ci-dessus, caractérisée en ce que ladite levure sous forme sèche a une force fermentative inférieure à 70 ml dans une pâte sucrée à 20%.

Une composition préférée selon l'invention est ainsi une composition telle que définie ci-dessus, caractérisée en ce que ladite levure sous forme sèche a une force fermentative :
- inférieure à 70 ml dans une pâte sans sucre, de préférence inférieure à 60 ml dans une pâte sans sucre,
- inférieure à 90 ml dans une pâte sucrée à 10%, de préférence inférieure à 80 ml dans une pâte sucrée à 10%, et
- est inférieure à 70 ml dans une pâte sucrée à 20%.

La levure est caractérisée ici par une force fermentative mesurée sur levure sèche.

Toutefois, dans la composition selon l'invention, la levure peut être utilisée sous toute forme, par exemple une levure liquide, une levure pressée ou une levure sèche.

La levure liquide, la levure pressée et la levure sèche sont telles que définies ci-dessus. Comme indiqué précédemment, la présente invention permet de remplacer totalement la levure de boulangerie traditionnelle par une seule levure qui est utilisée à la fois comme agent levant et agent de masquage de la note levure.

La levure selon la description peut donc se substituer totalement à la levure de boulangerie traditionnelle et il n'est pas nécessaire d'ajouter une levure de boulangerie traditionnelle en supplément de la levure selon la description.

Une composition préférée selon l'invention est une composition telle que définie ci-dessus caractérisée en ce qu'elle ne comprend pas de levure de boulangerie traditionnelle, et plus généralement ne comprend pas de levure habituellement utilisée en panification.

Ainsi, une composition préférée selon l'invention est une composition telle que définie ci-dessus apte à donner un produit de panification, comprenant une levure, de la farine, et du sel, caractérisée en ce que ladite levure a une force fermentative sous forme sèche inférieure à 70 ml dans une pâte sans sucre et en ce que ladite levure constitue l'agent levant.

L'expression « ladite levure constitue l'agent levant » signifie que ladite levure est la seule source d'agent levant de la composition.

Une levure préférée selon la description est une levure telle que définie ci-dessus qui est également capable de produire à 35°C au moins 13° d'alcool Gay-Lussac, de préférence au moins 13,5°, dans un milieu synthétique comprenant du sucre en quantité non limitante, une source d'azote, une source de phosphore, ainsi que les vitamines et minéraux essentiels à la croissance d'une levure.

Une telle levure est par exemple une levure obtenue par culture de la souche de levure I-4445. Un tel milieu synthétique comprend par exemple 265 g/kg de glucose, 2,47 g/kg d'acétate d'ammonium, 0,72 g/kg de potassium dihydrogénophosphate, 0,3 g/kg d'ammoniaque, 5 g/kg d'extrait de levure, 10 mg/kg d'inositol, 4 mg/kg de vitamine B1, 4 mg/kg de vitamine B6, 40 mg/kg d'acide nicotinique, 0,004 mg/kg de biotine, le pH étant ajusté à 5,5.

Une autre levure préférée selon la description est une levure telle que définie ci-dessus et dont le profil fermentaire sur 24h de fermentation à 30°C dans une pâte sans sucre obtenue selon le protocole pour la mesure de la force fermentative, mais avec 320 mg de matières sèches levure, présente l'ensemble des caractéristiques suivantes :
- un premier pic de vitesse de dégagement gazeux pendant la première heure de fermentation,
- une vitesse de dégagement gazeux à 2 heures inférieure à 0,30 ml/min,
- un cumul de dégagement gazeux à 2 heures inférieur à 90 ml, et
- un deuxième pic de vitesse de dégagement gazeux dans un délai supérieur à 8 heures ou aucun deuxième pic.

C'est le cas notamment de la levure obtenue par culture de la souche de levure I-4446 ou de la souche de levure I-4447 (cf. figure 1).

Les levures de brasserie et les levures de boulangerie traditionnelles ont un profil fermentaire totalement différent (cf. figure 1).

La présente invention a pour objet une composition telle que définie ci-dessus, caractérisée en ce qu'elle comprend au moins 0,9% de ladite levure, le pourcentage étant exprimé en masse de matière sèche sur la masse de farine.

La présente invention a particulièrement pour objet une composition telle que définie ci-dessus, caractérisée en ce qu'elle comprend de la farine, du sel, et au moins 0,9% de ladite levure masquant totalement ou partiellement la note levure, ladite levure ayant une force fermentative sous forme sèche inférieure à 70ml dans une pâte sans sucre.

La force fermentative est mesurée tel que défini ci-dessus.

La présente invention a également pour objet une composition telle que définie ci-dessus, caractérisée en ce qu'elle comprend par exemple au moins 1,3% de ladite levure, le pourcentage étant exprimé en masse de matière sèche sur la masse de farine.

Une composition préférée selon l'invention est une composition telle que définie ci-dessus, caractérisée en ce qu'elle comprend de 0,9% à 3,2% de ladite levure, de préférence de 1,1% à 1,9% de ladite levure, le pourcentage étant exprimé en masse de matière sèche sur la masse de farine.

La composition telle que définie ci-dessus selon l'invention peut comprendre au moins deux levures différentes obtenues à partir de souches de levure selon l'invention différentes. Toutefois, une composition telle que définie ci-dessus selon l'invention comprend une seule sorte de levure issue d'une souche de levure selon la description.

La présente invention a plus particulièrement pour objet une composition telle que définie ci-dessus, caractérisée en ce que ladite levure est obtenue par culture d'une souche de levure obtenue par le procédé de sélection tel que défini ci-dessus ou une souche dérivée d'une souche de levure obtenue par ledit procédé de sélection.

La présente invention a encore plus particulièrement pour objet une composition telle que définie ci-dessus, caractérisée en ce que ladite levure est obtenue par culture de la souche de levure I-4445, la souche de levure I-4446, la souche de levure I-4447 ou une souche dérivée desdites souches de levure.

La présente invention a plus particulièrement pour objet une composition telle que définie ci-dessus, caractérisée en ce que ladite levure est obtenue par culture de la souche de levure I-4445, la souche de levure I-4446, la souche de levure I-4447, une autre souche de levure obtenue par le procédé de sélection tel que défini ci-dessus ou une souche dérivée desdites souches de levure.

Une souche de levure obtenue par le procédé de sélection tel que défini ci-dessus et utilisée dans la composition ci-dessus n'est pas une souche de levure de boulangerie traditionnelle, et plus généralement n'est pas une souche de levure utilisée en panification.

La présente invention a également pour objet une composition telle que définie ci-dessus, caractérisée en ce qu'elle comprend moins de 90% d'eau, de préférence moins de 80% d'eau, plus préférentiellement moins de 70% d'eau, le pourcentage étant exprimé en masse sur la masse de farine.

Lorsque la composition selon l'invention comprend de l'eau, les différents constituants sont de préférence mélangés afin d'obtenir une composition homogène.

La composition selon l'invention est alors une pâte.

La présente invention a ainsi particulièrement pour objet une composition telle que définie ci-dessus, caractérisée en ce qu'elle est sous la forme d'une pâte non fermentée, une pâte fermentée, une pâte crue surgelée, une pâte fermentée surgelée, une pâte précuite ou une pâte précuite surgelée.

Une pâte non fermentée est une pâte obtenue par mélange des ingrédients, et plus précisément par pétrissage.

Une pâte fermentée est une pâte obtenue après l'étape de fermentation appelée « fermentation finale » ou « apprêt ».

Une pâte crue surgelée désigne ici une pâte qui est surgelée avant l'étape de fermentation finale, mais après l'étape de pointage.

Une pâte précuite est une pâte fermentée partiellement cuite.

Une pâte précuite surgelée est une pâte fermentée partiellement cuite avant d'être surgelée.

La présente invention a pour objet une composition telle que définie ci-dessus, caractérisée en ce qu'elle est sous forme sèche.

L'expression « sous forme sèche » signifie que la composition comprend au moins 85% de matière sèches, de préférence au moins 90% de matières sèches, plus préférentiellement au moins 95% de matières sèches, le pourcentage étant exprimé en masse sur la masse de la composition.

Une composition selon l'invention sous forme sèche est de préférence une préparation prête à l'emploi.

Une préparation prête à l'emploi est une composition qui comprend tous les ingrédients du produit de panification, excepté l'eau et éventuellement un autre liquide tel que le lait.

La présente invention a également pour objet une composition telle que définie ci-dessus, caractérisée en ce qu'elle comprend du sucre, de préférence au moins 5% de sucre, plus préférentiellement au moins 10% de sucre, le pourcentage étant exprimé en masse sur la masse de farine.

Une composition selon l'invention peut comprendre au moins 20% de sucre, le pourcentage étant exprimé en masse sur la masse de farine.

Le sucre est apporté par exemple sous la forme de saccharose, glucose, fructose, ou un mélange de ceux-ci.

La présente invention a également pour objet une composition telle que définie ci-dessus caractérisée en ce qu'elle comprend des matières grasses, du lait, des oeufs, et/ou un améliorant.

Le lait est par exemple du lait en poudre ou sous forme liquide.

Les matières grasses sont par exemple de la margarine, de l'huile, du beurre, un dérivé de ceux-ci et/ou un mélange de ceux-ci.

La présente invention a par exemple pour objet une composition telle que définie ci-dessus caractérisée en ce qu'elle comprend au moins 5% de matières grasses, de préférence au moins 10% de matières grasses, plus préférentiellement encore au moins 15% de matières grasses, le pourcentage étant exprimé en masse sur la masse de farine.

Un améliorant contient par exemple au moins un oxydant et/ou au moins un réducteur et/ou au moins une enzyme et/ou au moins un émulsifiant.

Des compositions préférées selon l'invention figurent dans les tableaux 2, 3, 5 ou 7 de l'exemple 2, ainsi que ces mêmes compositions sans l'ingrédient eau.

La présente invention a également pour objet un procédé de préparation d'une composition apte à donner un produit de panification telle que définie ci-dessus, comprenant une étape de mélange des ingrédients comprenant une levure, obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447, de la farine et du sel, ladite levure ayant une force fermentative sous forme sèche inférieure à 70 ml dans une pâte sans sucre. Ladite levure selon la description est telle que définie ci-dessus, obtenue par culture d'une souche de levure selon la description.

La souche de levure est une souche de levure telle que définie ci-dessus.

La force fermentative est mesurée dans une pâte contenant 20g de farine sur une durée de 2 heures à 30°C, tel que défini ci-dessus.

La présente invention a particulièrement pour objet un procédé de préparation d'une composition apte à donner un produit de panification telle que définie ci-dessus, comprenant une étape de mélange des ingrédients comprenant de la farine, du sel et au moins 0,9% d'une levure masquant totalement ou partiellement la note levure, le pourcentage étant exprimé en masse de matière sèche sur la masse de farine, ladite levure ayant une force fermentative sous forme sèche inférieure à 70 ml dans une pâte sans sucre.
et obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447.

Plus généralement, les ingrédients sont tels que définis ci-dessus, dans les quantités telles que définies ci-dessus et peuvent comprendre d'autres ingrédients tels que définis ci-dessus. L'étape de mélange correspond à l'étape de pétrissage lorsque la composition apte à donner un produit de panification est une pâte.

La présente invention a également pour objet un procédé de préparation tel que défini ci-dessus d'un produit de panification comprenant les étapes suivantes :
a) une étape de mélange des ingrédients comprenant une levure selon l'invention, farine, sel et eau, pour obtenir une pâte,
b) une étape de fermentation de la pâte, pour obtenir une pâte fermentée, et
c) une étape de cuisson de la pâte fermentée, pour obtenir un produit de panification.

La levure selon l'invention est telle que définie ci-dessus.

La présente invention a plus particulièrement pour objet un procédé de préparation tel que défini ci-dessus d'un produit de panification comprenant les étapes suivantes :
a) une étape de mélange des ingrédients comprenant une levure, farine, sel et eau, ladite levure étant obtenue par culture d'une souche de levure selon l'invention, pour obtenir une pâte,
b) une étape de fermentation de la pâte, pour obtenir une pâte fermentée, et
c) une étape de cuisson de la pâte fermentée pour obtenir un produit de panification.

Une souche de levure selon l'invention est telle que définie ci-dessus.

La présente invention a particulièrement pour objet un procédé de préparation d'un produit de panification comprenant les étapes suivantes :
a) une étape de mélange des ingrédients comprenant une obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447, farine, sel et eau, ladite levure ayant une force fermentative sous forme sèche inférieure à 70 ml dans une pâte sans sucre, pour obtenir une pâte,
b) une étape de fermentation de la pâte, pour obtenir une pâte fermentée, et
c) une étape de cuisson de la pâte fermentée pour obtenir un produit de panification.

La force fermentative est mesurée tel que défini ci-dessus.

La présente invention a particulièrement pour objet un procédé de préparation d'un produit de panification comprenant les étapes suivantes :
a) une étape de mélange des ingrédients comprenant farine, sel, eau et au moins 0,9% d'une levure masquant totalement ou partiellement la note levure, le pourcentage étant exprimé en masse de matière sèche sur la masse de farine, ladite levure ayant une force fermentative sous forme sèche inférieure à 70 ml dans une pâte sans et obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447, pour obtenir une pâte,
b) une étape de fermentation de la pâte, pour obtenir une pâte fermentée, et
c) une étape de cuisson de la pâte fermentée pour obtenir un produit de panification.

### L'étape de mélange a) correspond au pétrissage de la pâte.

L'étape de mélange a) peut être réalisée manuellement, dans un pétrin ou dans une machine à pain.

La pâte obtenue à l'issue de l'étape a) et la pâte fermentée obtenue à l'issue de l'étape b) ont de préférence les caractéristiques d'une composition apte à donner un produit de panification telle que définie ci-dessus.

L'étape de mélange a) peut consister à fournir une composition apte à donner un produit de panification telle que définie ci-dessus.

L'étape de mélange a) peut consister à mélanger une composition apte à donner un produit de panification telle que définie ci-dessus avec de l'eau.

Les étapes de mélange a) et de fermentation b) peuvent consister à fournir une composition apte à donner un produit de panification telle que définie ci-dessus.

Dans un mode de réalisation préféré selon l'invention, les ingrédients mélangés lors de l'étape a) ne comprennent pas un levain et/ou un starter et/ou une préparation obtenue après une phase de fermentation.

La présente invention a pour objet le procédé de préparation d'un produit de panification tel que défini ci-dessus, caractérisé en ce que les ingrédients comprennent au moins 0,9% de ladite levure, de préférence au moins 1,3% de ladite levure, le pourcentage étant exprimé en masse de matière sèche sur la masse de farine.

La présente invention a également pour objet le procédé de préparation d'un produit de panification tel que défini ci-dessus, caractérisé en ce que les ingrédients comprennent de 0,9% à 3,2% de ladite levure, de préférence de 1,1% à 1,9% de ladite levure, le pourcentage étant exprimé en masse de matière sèche sur la masse de farine.

La présente invention a plus particulièrement pour objet le procédé de préparation d'un produit de panification tel que défini ci-dessus, caractérisé en ce que la levure est obtenue par culture d'une souche de levure obtenue par le procédé de sélection tel que défini ci-dessus ou une souche dérivée d'une souche de levure obtenue par ledit procédé de sélection.

La présente invention a encore plus particulièrement pour objet le procédé de préparation d'un produit de panification tel que défini ci-dessus, caractérisé en ce que ladite levure est obtenue par culture de la souche de levure I-4445, la souche de levure I-4446, la souche de levure I-4447 ou une souche dérivée desdites souches de levure.

La présente invention a plus particulièrement pour objet le procédé de préparation d'un produit de panification tel que défini ci-dessus, caractérisé en ce que la levure est obtenue par culture de la souche de levure I-4445, la souche de levure I-4446, la souche de levure I-4447, une autre souche de levure obtenue par le procédé tel que défini ci-dessus ou une souche dérivée desdites souches de levure.

La présente invention a plus particulièrement pour objet le procédé de préparation d'un produit de panification tel que défini ci-dessus, caractérisé en ce que les ingrédients dans l'étape a) de mélange comprennent également au moins 5% de sucre, de préférence au moins 10% de sucre, le pourcentage étant exprimé en masse sur la masse de farine.

### L'étape b) de fermentation correspond à l'apprêt.

L'étape b) de fermentation est généralement précédée d'une 1^{ère} étape de fermentation appelée pointage.

L'étape b) de fermentation est donc appelée indifféremment 2^{ème} fermentation ou fermentation finale.

La présente invention a également pour objet un procédé de préparation d'un produit de panification tel que défini ci-dessus, caractérisé en ce qu'il comprend une étape de surgélation de la pâte obtenue à l'étape a) avant l'étape b) de fermentation.

L'étape de surgélation est de préférence effectuée après une étape de pointage.

La présente invention a également pour objet le procédé tel que défini ci-dessus dans lequel l'étape de cuisson est effectuée dans un four ou en machine à pain.

L'étape c) de cuisson n'est de préférence pas réalisée par cuisson à la vapeur, aux micro-ondes et/ou par friture.

La présente invention a particulièrement pour objet un procédé de préparation d'un produit de panification tel que défini ci-dessus, caractérisée en ce qu'au moins une des étapes du procédé est effectuée en machine à pain.

La présente invention a également pour objet un procédé de préparation d'un produit de panification tel que défini ci-dessus, caractérisé en ce qu'il comprend une étape de pré-cuisson et/ou de surgélation entre l'étape b) de fermentation et l'étape c) de cuisson.

La présente invention a particulièrement pour objet un procédé de préparation d'un produit de panification tel que défini ci-dessus comprenant les étapes suivantes :
- une étape de mélange des ingrédients comprenant une levure, farine, sel et eau, ladite levure ayant une force fermentative sous forme sèche inférieure à 70 ml dans une pâte sans sucre, pour obtenir une pâte,
- une étape de pointage,
- une éventuelle étape de surgélation,
- une étape de fermentation de la pâte, pour obtenir une pâte fermentée,
- une éventuelle étape de pré-cuisson,
- une éventuelle étape de surgélation, et
- une étape de cuisson de la pâte fermentée pour obtenir un produit de panification.

La présente invention a plus particulièrement pour objet un procédé de préparation d'un produit de panification tel que défini ci-dessus comprenant les étapes suivantes :
- une étape de mélange des ingrédients comprenant farine, sel, eau, et au moins 0,9% d'une levure masquant totalement ou partiellement la note levure, le pourcentage étant exprimé en masse de matière sèche sur la masse de farine, ladite levure ayant une force fermentative sous forme sèche inférieure à 70 ml dans une pâte sans sucre, pour obtenir une pâte,
- une étape de pointage,
- une éventuelle étape de surgélation,
- une étape de fermentation de la pâte, pour obtenir une pâte fermentée,
- une éventuelle étape de pré-cuisson,
- une éventuelle étape de surgélation, et
- une étape de cuisson de la pâte fermentée pour obtenir un produit de panification.

La présente invention concerne également l'utilisation d'une souche de levure pour obtenir un agent levant et de masquage de la note levure, caractérisée en ce que ladite souche de levure est choisie parmi la souche de levure I-4445, la souche de levure I-4446, la souche de levure I-4447, une souche de levure obtenue par le procédé de sélection tel que défini ci-dessus ou une souche dérivée desdites souches de levure.

Le masquage de la note levure est partiel ou total.

La présente invention a plus particulièrement pour objet l'utilisation telle que définie ci-dessus d'une souche de levure pour obtenir un agent levant et de masquage de la note levure destiné à des panifications utilisant une quantité importante de levure.

La présente invention a plus particulièrement pour objet l'utilisation telle que définie ci-dessus d'une souche de levure pour obtenir un agent levant et de masquage de la note levure destiné à des panifications utilisant au moins 0,9% de levure, de préférence 1,3% de levure, en masse de matière sèche sur la masse de farine.

La présente invention a plus particulièrement pour objet l'utilisation telle que définie ci-dessus d'une souche de levure pour obtenir un agent levant et de masquage de la note levure destiné à des panifications utilisant de 0,9% à 3,2% de levure, de préférence de 1,1% à 1,9% de levure, en masse de matière sèche sur la masse de farine.

La présente invention a plus particulièrement pour objet l'utilisation telle que définie ci-dessus d'une souche de levure pour obtenir un agent levant et de masquage de la note levure destiné à des panifications utilisant au moins 5% de sucre, de préférence au moins 10% de sucre, en masse sur la masse de farine.

La présente invention a plus particulièrement pour objet l'utilisation telle que définie ci-dessus d'une souche de levure pour obtenir un agent levant et de masquage de la note levure destiné à des applications ménagères.

La présente invention a plus particulièrement pour objet l'utilisation telle que définie ci-dessus d'une souche de levure pour obtenir un agent levant et de masquage de la note levure destiné à des panifications en pâte crue surgelée.

La présente invention a également pour objet l'utilisation telle que définie ci-dessus d'une levure dont la force fermentative sous forme sèche dans une pâte sans sucre est inférieure à 70 ml, comme agent levant et de masquage de la note levure.

La force fermentative est mesurée tel que défini ci-dessus.

Le masquage de la note levure est partiel ou total.

La présente invention a particulièrement pour objet l'utilisation telle que définie ci-dessus d'une levure dont la force fermentative sous forme sèche dans une pâte sans sucre est inférieure à 70 ml, comme agent levant et de masquage de la note levure dans une composition apte à donner un produit de panification.

La présente invention a plus particulièrement pour objet l'utilisation telle que définie ci-dessus d'une levure, caractérisée en ce que la levure est obtenue par culture d'une souche de levure obtenue par le procédé de sélection tel que défini ci-dessus ou une souche dérivée d'une souche de levure obtenue par ledit procédé de sélection.

La présente invention a encore plus particulièrement pour objet l'utilisation telle que définie ci-dessus d'une levure, caractérisée en ce que ladite levure est obtenue par culture de la souche de levure I-4445, la souche de levure I-4446, la souche de levure I-4447 ou une souche dérivée desdites souches de levure.

La présente invention a plus particulièrement pour objet l'utilisation telle que définie ci-dessus d'une levure, caractérisée en ce que levure est obtenue par culture de la souche de levure I-4445, la souche de levure I-4446, la souche de levure I-4447, une autre souche de levure obtenue par le procédé de sélection tel que défini ci-dessus ou une souche dérivée desdites souches de levure.

La présente invention a plus particulièrement pour objet l'utilisation telle que définie ci-dessus d'une levure comme agent levant et de masquage de la note levure destiné à des panifications utilisant une quantité importante de levure.

La présente invention a plus particulièrement pour objet l'utilisation telle que définie ci-dessus d'une levure comme agent levant et de masquage de la note levure destiné à des panifications utilisant au moins 0,9% de levure, en masse de matière sèche sur la masse de farine.

La présente invention a plus particulièrement pour objet l'utilisation telle que définie ci-dessus d'une levure pour obtenir un agent levant et de masquage de la note levure destiné à des panifications utilisant de 0,9% à 3,2% de levure, de préférence de 1,1% à 1,9% de levure, le pourcentage étant exprimé en masse de matière sèche sur la masse de farine.

La présente invention a plus particulièrement pour objet l'utilisation telle que définie ci-dessus d'une levure comme agent levant et de masquage de la note levure destiné à des panifications utilisant au moins 5% de sucre, de préférence au moins 10% de sucre, le pourcentage étant exprimé en masse sur la masse de farine.

La présente invention a plus particulièrement pour objet l'utilisation telle que définie ci-dessus d'une levure comme agent levant et de masquage de la note levure destiné à des applications ménagères.

La présente invention a plus particulièrement pour objet l'utilisation telle que définie ci-dessus d'une levure comme agent levant et de masquage de la note levure destiné à des panifications en pâte crue surgelée.

Les exemples suivants illustrent l'invention sans la limiter.

Les exemples décrivent en particulier la force fermentative des levures selon la description et le masquage de la note levure obtenue grâce aux levures selon la description dans différents recettes et protocoles de panification.

### EXEMPLE 1: CARACTERISATION DE LA FORCE FERMENTATIVE DES LEVURES SELON L'INVENTION

### Matériel et Méthodes

### (i) Production de levures sèches

Dans toutes les recettes suivantes, les levures utilisées sont sous la forme de levures sèches. Les levures sont produites à partir des souches de levure selon l'invention dans des fermenteurs, comme décrit dans le livre de référence « Yeast Technology », 2ème édition, 1991, G. Reed et T.W. Nagodawithana, publié par Van Nostrand Reinhold, ISBN 0-442-31892-8. Les levures sont ensuite séchées pour obtenir des levures sèches.

### (ii) Mesure des forces fermentatives

La force fermentative des levures est mesurée sur levure sèche, dans une pâte sans sucre et dans des pâtes sucrées à 10% et 20% (pourcentages exprimés en masse sur la masse de farine).

La pâte contient 20g de farine, auxquels 2g de saccharose sont ajoutés pour la pâte sucrée à 10% et 4g de saccharose pour la pate sucrée à 20%.

La levure sèche, dans une quantité égale à 160 mg de matières sèches, est réhydratée dans 6 ml d'eau distillée à 38°C pendant 15 minutes. On ajoute ensuite 9 ml d'un mélange d'eau et 405 mg de NaCl. On obtient ainsi une suspension de levures dans une solution aqueuse contenant 27 g/l de NaCl.

La farine (avec ou sans saccharose) et ladite suspension de levures sont malaxées pendant 40 secondes dans un pétrin, de manière à obtenir une pâte qui est ensuite placée dans un récipient mis au bain-marie à 30°C. 13 minutes après le début du malaxage, le récipient contenant la pâte est fermé hermétiquement.

Le volume de dégagement gazeux est mesuré au cours de la 1^{ère} heure et au cours de la 2^{ème} heure, au moyen d'un Risograph (*National Manufacturing, Lincoln, Nebraska*). On indique ensuite le volume total en ml sur 2 heures à 30°C.

### Résultats

La force fermentative des levures selon l'invention figure dans le tableau 1 (dégagement gazeux en ml).

A titre de comparaison, la force fermentative obtenue avec deux levures témoin sont indiquées. T1 est une levure adaptée à une utilisation dans des pâtes sucrées et T2 dans des pâtes sans sucre.

**Tableau 1**

| **Souche de levure** | **Pâte sans sucre** | **Pâte sucrée 10%** | **Pâte sucrée 20%** |
|---|---|---|---|
| I-4445 | 50 | 60 | 41 |
| I-4446 | 35 | 72 | 55 |
| I-4447 | 44 | 73 | 62 |
| T1 | 76 | 101 | 115 |
| T2 | 112 | 94 | 67 |

Les levures selon l'invention ont un profil fermentaire bien particulier : dans une pâte sans sucre, leur force fermentative est inférieure à 60 ml, soit bien inférieure à la fois à celle d'une levure adaptée aux pâtes sans sucre, mais également à celle d'une levure adaptée aux pâtes sucrées et qui n'est donc pas très performante sur pâte sans sucre.

La force fermentative des levures selon l'invention dans une pâte sucrée à 10% est inférieure à 80 ml, soit bien inférieure à celle d'une levure adaptée aux pâtes sucrées, mais également à celle d'une levure adaptée aux pâtes sans sucre et qui n'est donc pas très performante sur pâte sans sucre.

Enfin, dans une pâte sucrée à 20%, la force fermentative des levures selon l'invention est inférieure à 70 ml, soit bien inférieure à celle d'une levure adaptée aux pâtes sucrées. Toutefois, l'écart entre la force fermentative des levures selon l'invention et celle d'une levure adaptée à une pâte sans sucre est moins important dans une pâte sucrée à 20%.

### EXEMPLE 2: DIMINUTION DE LA NOTE LEVURE OBTENUE GRACE AUX LEVURES SELON L'INVENTION DANS DIFFERENTES RECETTES ET DIFFERENTS PROTOCOLES DE PANIFICATION

### Matériel et Méthodes

### (i) Levures

Les levures testées sont sous forme de levure sèche.

### (ii) Pain brioché obtenu en machine à pain

La recette du pain brioché (*cf*. *tableau 2*) est une recette contenant 15,2% de sucre (pourcentage exprimé par rapport à la masse de farine). Elle est mise en oeuvre en machine à pain (référence : Moulinex Homebread).

Le programme « brioche » est utilisé avec les paramètres suivants :
- poids du pain : 750g,
- intensité de cuisson : 1.
Sont introduits dans la cuve de la machine à pain :
a) eau,
b) sel, sucre, lait en poudre, margarine,
c) farine, et
d) levure.

Le programme est démarré en appuyant sur le bouton « start ».

A la fin du programme, le pain brioché est sorti de la machine à pain, puis refroidi sur une grille.

Le pain brioché non tranché est emballé dans un sac plastique.

La levure témoin (Bruggeman marron) est une levure de boulangerie classiquement utilisée pour ce type de panification.

**Tableau 2**

| **Ingrédients** | **Quantité en gramme** | **Quantité en % du boulanger** |
|---|---|---|
| Farine | 500 | 100 |
| Eau | 270 | 54 |
| Sucre | 76 | 15,2 |
| Lait en poudre | 24 | 4,8 |
| Sel | 8 | 1,6 |
| Margarine | 75 | 15 |
| Levure sèche | 7,5 (5 pour le témoin) | 1,5 (1 pour le témoin) |

### (iii) Baguette en schéma direct

La baguette en schéma direct est une baguette obtenue selon un schéma très court, en seulement 2h.

La recette est indiquée dans le tableau 3 et le protocole appliqué dans le tableau 4.

La levure témoin (Bruggeman bleu) est une levure de boulangerie classiquement utilisée pour ce type de panification.

L'améliorant apporte le mélange d'oxydants et de réducteurs, les enzymes ainsi que les émulsifiants classiques permettant une optimisation du processus de fabrication, une bonne qualité et une bonne conservation des pains obtenus.

**Tableau 3**

| **Ingrédients** | **Quantité en gramme** | **Quantité en % du boulanger** |
|---|---|---|
| Farine | 1500 | 100 |
| Eau | 990 | 66 |
| Sel | 27 | 1,8 |
| Levure sèche | 27 (19,5 pour le témoin) | 1,8 (1,3 pour le témoin) |
| Améliorant (Ibis bleu) | 7,5 | 0,5 |

**Tableau 4**

| | |
|---|---|
| Pétrin | spiral |
| Pétrissage 1^{ère} vitesse | 4 min |
| Pétrissage 2^{ème} vitesse | 3 min + 1 min |
| Pointage (1^{ère} fermentation) | 30 min |
| Division | 330 g |
| Boulage | |
| Détente | 10 min |
| Façonnage | Peu serré |
| Apprêt (2^{ème} fermentation) | 45 min à 22°C |
| Cuisson en four à sole | Enfournement à 250°C, cuisson 22 min à 230°C |

### (iv) Brioche obtenue à partir d'une pâte crue surgelée

La brioche est obtenue à partir d'une pâte surgelée avant la fermentation.

La recette est indiquée dans le tableau 5 et le protocole appliqué dans le tableau 6.

La levure témoin (Bruggeman marron) est une levure de boulangerie classiquement utilisée pour ce type de panification.

**Tableau 5**

| **Ingrédients** | **Quantité en gramme** | **Quantité en % du boulanger** |
|---|---|---|
| Farine | 1500 | 100 |
| Eau | 525 | 35 |
| Sel | 30 | 2 |
| Levure | 45 | 3 (2 pour le témoin) |
| Améliorant (Ibis Violet) | 12 | 0,8 |
| Sucre | 180 | 12 |
| Beurre | 375 | 25 |
| Oeufs | 375 | 25 |

L'améliorant apporte le mélange d'oxydants et de réducteurs, les enzymes ainsi que les émulsifiants classiques permettant une optimisation du processus de fabrication, une bonne qualité et une bonne conservation des pains obtenus.

La brioche non tranchée est emballée dans un sac plastique.

**Tableau 6**

| Pétrin | spiral |
|---|---|
| Pétrissage 1^{ère} vitesse | 5 min |
| Pétrissage 2^{ème} vitesse | 5 min |
| Température de la pâte | 25°C |
| Pointage (1^{ère} fermentation) | 10 min |
| Division | 50 g |
| Surgélation | 15 min à -30°C |
| Décongélation | 15h à 4°C ou 2h à température ambiante |
| Apprêt (2^{ème} fermentation) | 90 min |
| Cuisson | 12 min à 190°C |

### (v) Rotimani

Le rotimani est un pain de mie indonésien obtenu à partir d'une pâte contenant une teneur en sucre très élevée (23% en masse sur la masse de farine).

La recette est indiquée dans le tableau 7 et le protocole appliqué dans le tableau 8.

La levure témoin (Bruggeman marron) est une levure de boulangerie classiquement utilisée pour ce type de panification.

**Tableau 7**

| **Ingrédients** | **Quantité en gramme** | **Quantité en % du boulanger** |
|---|---|---|
| Farine | 800 | 100 |
| Eau | 360 | 45 |
| Sel | 12 | 1,5 |
| Levure sèche | 27,6 (18,4 pour le témoin) | 3,45 (2,3 pour le témoin) |
| Sucre | 184 | 23 |
| Shortening Wilmar (matière grasse) | 80 | 10 |
| Améliorant Ibis violet | 2,4 | 0,3 |
| Propionate de calcium | 2,4 | 0,3 |
| Poudre de lait | 24 | 3 |
| Oeufs | 80 | 10 |

L'améliorant apporte le mélange d'oxydants et de réducteurs, les enzymes ainsi que les émulsifiants classiques permettant une optimisation du processus de fabrication, une bonne qualité et une bonne conservation des pains obtenus.

Le rotimani non tranché est emballé dans un sac plastique.

**Tableau 8**

| Pétrin | à aiguilles |
|---|---|
| Pétrissage 1^{ère} vitesse | 1 min avant l'ajout des matières grasses, 1 min d'arrêt pour incorporer les matières grasses, puis 1 min |
| Pétrissage 2^{ème} vitesse | 5 min 30 |
| Pointage (1^{ère} fermentation) | 10 min |
| Détente | 10 min |
| Façonnage | |
| Apprêt (2^{ème} fermentation) | 90 min à 35°C |
| Cuisson | 21 min à 190°C |

### (vi) Analyse sensorielle

L'évaluation sensorielle des produits de panification (ii) à (v) est effectuée par un panel d'experts de 12 à 15 personnes.

Les experts attribuent une note entre 0 et 10 pour chaque descripteur et chaque produit de panification.

La moyenne des notes est ensuite réalisée. L'écart type dans la notation est également contrôlé, pour s'assurer du consensus entre les experts.

Les experts sont des sujets qualifiés qui ont une bonne acuité sensorielle et entraînés à l'utilisation des méthodes d'évaluation sensorielle et qui sont capables d'effectuer de façon fiable tous les types d'essais. Les performances des experts sont contrôlées régulièrement. Des entraînements sont effectuées pour maintenir le niveau du panel : une à deux séances d'une heure ont lieu par semaine, qui se déroulent dans des conditions répondant aux normes AFNOR, afin d'apprendre à décrire et noter précisément chaque descripteur entre 0 (référent faible) et 10 (référent dominant) pour n'importe quel pain des univers produits définis au départ.

Les panélistes travaillent dans des boxes individuels de dégustation, dans une salle spéciale ventilée. Un logiciel spécialisé est disponible pour la création des questionnaires, la saisie des données et l'exploitation statistiques des résultats.

### Résultats

Les recettes et protocoles de panification choisis se caractérisent par la nécessité d'utiliser une quantité importante de levure. Les levures utilisées de manière traditionnelle dans ce type de recettes et protocoles sont responsables de l'apparition d'une note levure sur les produits de panification ainsi obtenus.

Il s'agit par exemple des produits suivants :
- pain brioché (15,2% de sucre) obtenu en machine à pain,
- baguette en schéma direct obtenue selon un schéma très court (2h),
- brioche obtenue à partir d'une pâte crue surgelée, et
- rotimani, c'est-à-dire un pain de mie obtenu à partir d'une pâte contenant une très forte teneur en sucre (23%).

Les produits de panification obtenus avec les levures issues des souches selon l'invention (I-4445, I-4446 et I-4447) sont comparés à ceux obtenus avec une levure témoin qui est à chaque fois une levure de boulangerie de référence pour ce type de recette et protocole.

Le seul paramètre qui diffère avec les produits de panification obtenus avec la levure témoin est la nature et quantité de levure utilisée : la quantité de levure testée est en effet ajustée de manière à atteindre un pouvoir ferment équivalent à la levure témoin et permettre d'obtenir des produits de panification similaires à ceux obtenus avec la levure témoin, notamment en terme de volume spécifique.

L'écart entre le volume spécifique des produits de panification testés et celui du produit de panification témoin est toujours inférieur ou égal à + ou - 9%.

Les produits de panification obtenus sont ensuite évalués par un panel expert constitué de 12 à 15 personnes qui évalue chaque produit de panification en attribuant une note (de 0 à 10) par rapport aux descripteurs d'une liste de référence. La moyenne des notes est indiquée dans les tableaux 9 et 10.

Les résultats concernant la note levure sont résumés dans le tableau 9.

Malgré une dose de levure utilisée plus importante que pour la levure témoin, les levures issues des souches selon l'invention permettent de diminuer la note levure des produits de panification, à savoir diminuer l'odeur « levure » et l'arôme « levure » dans les différentes recettes et protocoles testés.

**Tableau 9**

| **Souche à l'origine de la levure testée** | **Pain brioché en machine à pain** | | **Baquette en schéma direct** | | **Brioche obtenue à partir d'une pâte crue surgelée** | | **Rotimani** | |
|---|---|---|---|---|---|---|---|---|
| | O | A | O | A | O | A | O | A |
| I-4445 | 2,25 | 1,77 | 3,58 | 2,75 | 2,75 | 2,05 | 2,15 | * |
| I-4446 | 2,17 | 2,38 | 3,58 | 2,86 | 1,56 | 2,13 | 2,40 | * |
| I-4447 | 1,67 | 1,77 | 3,29 | 1,43 | 2,09 | 2,11 | 2,94 | * |
| Témoin | 3,71 | 2,66 | 5,72 | 3,06 | 2,53 | 2,30 | 3,34 | * |

| | | | | | | | | |
|---|---|---|---|---|---|---|---|---|
| *O : odeur levure l fermenté - A : arôme levure* /*fermenté -* * *L'arôme levure n'est pas testé pour le rotimani, car le sucre présent en forte concentration masque l'arôme levure* | | | | | | | | |

Par ailleurs, des notes positives ressortent des produits de panification obtenus avec les levures selon l'invention, à un degré d'intensité nettement perceptible pour les experts (cf. dans le tableau 10).

**Tableau 10**

| | **Levure issue 1-4445** | **Levure issue 1-4446** | **Levure issue 1-4447** | **Levure témoin** |
|---|---|---|---|---|
| **Pain brioché en machine à pain** | | | | |
| odeur amande | **3,56** | 2,42 | **2,79** | 1,18 |
| arôme amande | **2,18** | 1,99 | **3,13** | 0,83 |
| odeur rhum | 1,35 | **2,58** | 1,55 | 1,77 |
| arôme rhum | 0,65 | **2,10** | 1,83 | 0,69 |

| **Baguette en schéma direct** | | | | |
|---|---|---|---|---|
| odeur blé mur | **4,29** | 1,43 | **4,29** | 1,43 |
| arôme blé mur | **4,29** | 1,43 | **4,29** | 1,43 |

| **Brioche obtenue à partir d'une pâte crue surgelée** | | | | |
|---|---|---|---|---|
| odeur rhum | **1,96** | **1,94** | 1,10 | 1,02 |
| arôme rhum | **1,10** | **1,59** | 0,68 | 0,46 |

| **Rotimani** | | | | |
|---|---|---|---|---|
| arôme rhum | **1,35** | **1,70** | **1,38** | 0,49 |
| saveur sucrée | **6,27** | **6,04** | **6,63** | 5,69 |

Les notes positives sont résumées par levure et type de recette/protocole dans le tableau 11.

**Tableau 11**

| **Souche à l'origine de la levure testée** | **Pain brioché en machine à pain** | **Baquette en schéma direct** | **Brioche obtenue à partir d'une pâte crue surgelée** | **Rotimani** |
|---|---|---|---|---|
| I-4445 | odeur et arôme amande | odeur et arôme blé mur | odeur et arôme rhum | arôme rhum saveur sucrée |
| I-4446 | odeur et arôme rhum | | odeur et arôme rhum | arôme rhum saveur sucrée |
| I-4447 | odeur et arôme amande | odeur et arôme blé mur | | arôme rhum saveur sucrée |

Les notes positives diffèrent selon la recette et le protocole utilisés.

Ainsi, les levures issues de la souche I-4445 et de la souche I-4447 sont très intéressantes pour la fabrication de produits de panifications en pâte sans sucre et sur un schéma court, par exemple pour la fabrication de baguettes en schéma direct.

Les levures issues de la souche I-4445 et de la souche I-4446 sont très intéressantes pour la fabrication d'un produit de panification sur pâte sucrée et/ou sur pâte crue surgelée, par exemple pour la fabrication d'une brioche obtenue à partir d'une pâte crue surgelée.

Les levures issues de la souche I-4445, de la souche I-4446 et de la souche I-4447 sont très intéressantes pour la fabrication de produits de panification sur pâte sucrée et/ou selon un protocole machine à pain, par exemple pour la fabrication d'un pain brioché obtenu en machine pain.

## Revendications

1. Composition apte à donner un produit de panification, comprenant de la farine, du sel, et au moins 0,9% d'une levure masquant totalement ou partiellement la note levure, le pourcentage étant exprimé en masse de matière sèche sur la masse de farine, ladite levure étant obtenue par culture d'une souche de levure sélectionnée parmi la souche de levure déposée le 9 février 2011 à la CNCM sous le numéro I-4445, la souche de levure déposée le 9 février 2011 à la CNCM sous le numéro I-4446, la souche de levure déposée le 9 février 2011 à la CNCM sous le numéro I-4447.

2. Composition selon la revendication 1, **caractérisée en ce qu'**elle comprend moins de 90% d'eau, le pourcentage étant exprimé en masse sur la masse de farine.

3. Composition selon la revendication 1 ou la revendication 2, **caractérisée en ce qu'**elle est sous forme sèche.

4. Composition selon l'une quelconque des revendications 1 à 3, **caractérisée en ce qu'**elle est sous la forme d'une pâte non fermentée, une pâte fermentée, une pâte crue surgelée, une pâte fermentée surgelée, une pâte précuite ou une pâte précuite surgelée.

5. Composition selon l'une quelconque des revendications 1 à 4, **caractérisée en ce qu'**elle comprend au moins 5% de sucre, le pourcentage étant exprimé en masse sur la masse de farine.

6. Procédé de préparation d'une composition apte à donner un produit de panification selon l'une quelconque des revendications 1 à 5, comprenant une étape de mélange des ingrédients comprenant de la farine, du sel, et au moins 0,9% d'une levure obtenue par culture d'une souche de levure définie dans la revendication 1, le pourcentage étant exprimé en masse de matière sèche sur la masse de farine.

7. Procédé de préparation d'un produit de panification comprenant les étapes suivantes :
a) une étape de mélange des ingrédients comprenant farine, sel, eau et au moins 0,9% d'une levure obtenue par culture d'une souche de levure définie dans la revendication 1 pour obtenir une pâte, le pourcentage étant exprimé en masse de matière sèche sur la masse de farine,
b) une étape de fermentation de la pâte, pour obtenir une pâte fermentée, et
c) une étape de cuisson de la pâte fermentée pour obtenir un produit de panification.

8. Procédé selon la revendication 7, **caractérisé en ce que** les ingrédients dans l'étape a) de mélange comprennent également au moins 5% de sucre, le pourcentage étant exprimé en masse sur la masse de farine.

9. Procédé selon la revendication 7 ou la revendication 8, **caractérisé en ce qu'**il comprend une étape de surgélation de la pâte obtenue à l'étape a), avant l'étape b) de fermentation.

10. Utilisation d'une levure obtenue par culture d'une souche de levure définie dans la revendication 1 comme agent levant et de masquage de la note levure dans une composition apte à donner un produit de panification.

## Patentansprüche

1. Zusammensetzung, die geeignet ist ein Brotprodukt zu ergeben, umfassend Mehl, Salz, und mindestens 0,9 % einer Hefe, die die Hefenote vollständig oder teilweise maskiert, wobei der Prozentgehalt ausgedrückt ist als Trockenmasse, bezogen auf die Mehlmasse, wobei die Hefe erhalten wird durch Kultivieren eines Hefestamms, ausgewählt aus dem Hefestamm, der am 9. Februar 2011 bei der CNCM unter der Nummer I-4445 hinterlegt wurde, dem Hefestamm, der am 9. Februar 2011 bei der CNCM unter der Nummer I-4446 hinterlegt wurde, dem Hefestamm, der am 9. Februar 2011 bei der CNCM unter der Nummer I-4447 hinterlegt wurde.

2. Zusammensetzung nach Anspruch 1, **dadurch gekennzeichnet, dass** sie mindestens 90 % Wasser enthält, wobei der Prozentgehalt als Masse, bezogen auf die Mehlmasse, angegeben ist.

3. Zusammensetzung nach Anspruch 1 oder Anspruch 2, **dadurch gekennzeichnet, dass** sie in trockener Form ist.

4. Zusammensetzung nach einem der Ansprüche 1 bis 3, **dadurch gekennzeichnet, dass** sie in der Form eines nichtfermentierten Teigs, eines fermentierten Teigs, eines rohen tiefgekühlten Teigs, eines tiefgekühlten fermentierten Teigs, eines vorgebackenen Teigs oder eines tiefgekühlten vorgebackenen Teigs ist.

5. Zusammensetzung nach einem der Ansprüche 1 bis 4, **dadurch gekennzeichnet, dass** sie mindestens 5 % Zucker enthält, wobei der Prozentgehalt als Masse, bezogen auf die der Mehlmasse, angegeben ist.

6. Verfahren zur Herstellung einer Zusammensetzung, die geeignet ist ein Brotprodukt nach einem der Ansprüche 1 bis 5 zu ergeben, umfassend einen Schritt des Mischens der Bestandteile, umfassend Mehl, Salz, und mindestens 0,9 % einer Hefe, erhalten durch Kultivieren einer Hefekultur, wie in Anspruch 1 definiert, wobei der Prozentgehalt ausgedrückt ist als Trockenmasse, bezogen auf die Mehlmasse.

7. Verfahren zur Herstellung eines Brotprodukts, umfassend die folgenden Schritte
a) einen Schritt des Mischens der Bestandteile, umfassend Mehl, Salz, Wasser und mindestens 0,9 % einer Hefe, erhalten durch Kultivieren einer Hefekultur, wie in Anspruch 1 definiert, zum Erhalten eines Teigs, wobei der Prozentgehalt ausgedrückt ist als Trockenmasse, bezogen auf die Mehlmasse;
b) einen Schritt des Fermentierens des Teigs, um einen fermentierten Teig zu erhalten; und
c) einen Schritt des Backens des fermentierten Teigs, um ein Brotprodukt zu erhalten.

8. Verfahren nach Anspruch 7, **dadurch gekennzeichnet, dass** die Bestandteile in Schritt a) des Mischens ebebfalls mindestens 5 % Zucker umfassen, wobei der Prozentgehalt als Masse, bezogen auf die Mehlmasse angegeben ist.

9. Verfahren nach Anspruch 7 oder 8, **dadurch gekennzeichnet, dass** es einen Schritt des Tiefkühlens des Teigs umfasst, der in Schritt a) erhalten wird, vor Schritt b) der Fermentation.

10. Verwendung einer Hefe, erhalten durch Kultivieren einer Hefekultur, wie in Anspruch 1 definiert, als Treibmittel und zur Maskierung der Hefenote in einer Zusammensetzung, die geeignet ist, um ein Brotprodukt zu ergeben.

## Claims

1. Composition capable of yielding a bakery product, comprising flour, salt, and at least 0.9% of a yeast that completely or partially masks the yeast note, the percentage being expressed as weight of dry matter relative to the weight of flour, said yeast being obtained by culture of a yeast strain selected from the yeast strain deposited on 9 February 2011 at the CNCM under number I-4445, the yeast strain deposited on 9 February 2011 at the CNCM under number I-4446, the yeast strain deposited on 9 February 2011 at the CNCM under number I-4447.

2. Composition according to Claim 1, **characterized in that** it comprises less than 90% of water, the percentage being expressed as weight relative to the weight of flour.

3. Composition according to Claim 1 or Claim 2, **characterized in that** it is in dry form.

4. Composition according to any one of Claims 1 to 3, **characterized in that** it is in the form of an unfermented dough, a fermented dough, a frozen raw dough, a frozen fermented dough, a precooked dough or a frozen precooked dough.

5. Composition according to any one of Claims 1 to 4, **characterized in that** it comprises at least 5% of sugar, the percentage being expressed as weight relative to the weight of flour.

6. Method of preparing a composition capable of yielding a bakery product according to any one of Claims 1 to 5, comprising a step of mixing ingredients comprising flour, salt, and at least 0.9% of a yeast obtained by culture of a yeast strain defined in Claim 1, the percentage being expressed as weight of dry matter relative to the weight of flour.

7. Method of preparing a bakery product comprising the following steps:
a) a step of mixing ingredients comprising flour, salt, water and at least 0.9% of a yeast obtained by culture of a yeast strain defined in Claim 1 to obtain a dough, the percentage being expressed as weight of dry matter relative to the weight of flour,
b) a step of fermentation of the dough, to obtain a fermented dough, and
c) a step of baking the fermented dough to obtain a bakery product.

8. Method according to Claim 7, **characterized in that** the ingredients in mixing step a) also comprise at least 5% of sugar, the percentage being expressed as weight relative to the weight of flour.

9. Method according to Claim 7 or Claim 8, **characterized in that** it comprises a step of deep-freezing the dough obtained in step a), before the fermentation step b).

10. Use of a yeast obtained by culture of a yeast strain defined in Claim 1 as leavening agent and agent masking the yeast note in a composition capable of yielding a bakery product.
